# EUROPEAN PATENT APPLICATION

(11) **EP 4 372 680 A1**
(43) Date of publication of application: **22.05.2024**
(21) Application number: 23205167.2
(22) Date of filing: 23.10.2023
(51) Int. Cl.: G06T 7/00, A61B 5/00

(54) **COMPUTER BASED CLINICAL DECISION SUPPORT SYSTEM AND METHOD FOR DETERMINING A CLASSIFICATION OF A LYMPHEDEMA INDUCED FLUORESCENCE PATTERN**

(30) Priority: 15.11.2022 US 202263425411 P
(71) Applicant: Quest Photonic Devices B.V., 1771 SR Wieringerwerf (NL)
(72) Inventor: HOVELING, Richelle Johanna Maria, 1623 JA Hoorn (NL); KOOPMAN, Thomas, 1013 WR Amsterdam (NL); MEESTER, Richard Johannes Cornelis, 1771 DA Wieringerwerf (NL)
(74) Representative: Seemann & Partner Patentanwälte mbB

(57) **Abstract**

Computer based clinical decision support system (CDSS) 62 and method for determining a classification of a lymphedema induced fluorescence pattern. The fluorescence image 7 is determined from a measurement of a fluorescence signal in a tissue of a body part 4, to which a fluorescent agent 8 has been added. The CDSS 62 comprising: an input interface 64 through which the fluorescence image 7, which is specific to a patient 6, is provided as an input feature to an artificial intelligence (Al) model 68, a processor 66, which performs an inference operation in which the fluorescence image 7 is applied to the Al model 68 to generate the classification of a lymphedema induced fluorescence pattern, and a user interface (UI) 14 through which the classification of the lymphedema induced fluorescence pattern is communicated to a user.

## Description

The invention relates to a computer based clinical decision support system (CDSS) that is configured to output a classification of a lymphedema induced fluorescence pattern based on a fluorescence image that is determined from a measurement of a fluorescence signal in a tissue of a body part, to which a fluorescent agent has been added. Furthermore, the invention relates to a computer implemented method of determining a classification of a lymphedema induced fluorescence pattern using a CDSS, the classification being based on a fluorescence image that is determined by measuring a fluorescence signal in a tissue of a body part, to which a fluorescent agent has been added.

Furthermore, the invention relates to a method of diagnosing lymphedema and to a method of long-term therapy of lymphedema.

Lymphedema is an accumulation of lymphatic fluid in the body's tissue. While oxygenated blood is pumped via the arteries from the heart to the tissue, deoxygenated blood returns to the heart via the veins. Because the pressure level on the arterial side is much higher than on the vein side, a colorless fluid part of the blood is pushed into the space between the cells. Typically, more fluid is pushed out, than reabsorbed on the vein side. The excess fluid is transported by the lymphatic capillaries. Furthermore, the fluid carries away local and foreign substances such as larger proteins and cellular debris. Once in the lymphatic system, this fluid including the transported substances is referred to as lymph or lymph fluid.

The lymphatic system comprises lymphatic vessels having one way valves similar to vein valves for transporting the lymph to the next lymph node. The lymph node performs removal of certain substances and cleans the fluid before it drains back to the blood stream.

If the lymphatic system becomes obstructed in that the lymph flow is blocked or not performed at the desired level, the lymph fluid accumulates in the interstitial space between the tissue cells. This accumulation, which is due to an impairment of lymphatic transport, is called lymphedema. The accumulation of lymph can cause inflammatory reaction which damages the cells surrounding the affected areas. It can further cause fibrosis which can turn into a hardening of the affected tissue.

Because lymphedema is a lifelong condition for that no cure or medication exists, early diagnoses and appropriate early counter measures for improving drainage and reducing the fluid load are of high importance for patients well-being and recovering. Possible treatments such as lymphatic massage and compression bandages up to surgery depend on the level of severity, which is a four stage system defined by the World Health Organization (WHO) as follows:

| | |
|---|---|
| Stage 1: | a normal flow in the lymphatic system. No signs or symptoms. |
| Stage 2: | accumulation of fluid with swelling. |
| Stage 3: | permanent swelling that does not resolve with elevation of the affected limb or body part. |
| Stage 4: | elephantiasis (large deformed limb), skin thickening with "wart like" growth and extensive scarring. |

For diagnosis of the function of the lymphatic system, commonly used techniques are a manual inspection of the affected limb or body part by a physician. A known imaging technique is lymphoscintigraphy. In this technique, a radio tracer is injected in the tissue of the affected body part and subsequently MRI (Magnetic Resonance Imaging), CT (Computer Tomography), a PET-CT-scan (Positron Emission Tomography) or ultrasound imaging is performed.

A relatively new imaging technique is infrared fluorescence imaging using a fluorescent dye, for example ICG (Indocyanine Green). ICG is a green colored medical dye that is used for over 40 years. The dye emits fluorescent light when exited with near infrared light having a wavelength between 600 nm and 800 nm. Due to this excitation, ICG emits fluorescence light between 750 nm and 950 nm. The fluorescence of the ICG dye can be detected using a CCD or CMOS sensor or camera. The fluorescent dye is administered to the tissue of an affected limb or body part and the concentration and flow of the lymphatic fluid can be traced on the basis of the detected fluorescence light.

It is an object of the invention to provide a computer based clinical decision support system (CDSS) for enhanced determination of a classification of a lymphedema induced fluorescence pattern and a computer implemented method of enhanced determination of a classification of a lymphedema induced fluorescence pattern.

Furthermore, it is an object of the invention to provide an enhanced method of diagnosing lymphedema and an enhanced method of long-term therapy of lymphedema.

The object is solved by a computer based clinical decision support system (in the following referred to as a "CDSS") that is configured to output a classification of a lymphedema induced fluorescence pattern based on a fluorescence image that is determined from a measurement of a fluorescence signal in a tissue of a body part, to which a fluorescent agent has been added, the CDSS comprising:
an input interface through which the fluorescence image, which is specific to a patient, is provided as an input feature to an artificial intelligence (Al) model,
a processor, which performs an inference operation in which the fluorescence image is applied to the AI model to generate the classification of a lymphedema induced fluorescence pattern, and
a user interface (III) through which the classification of the lymphedema induced fluorescence pattern is communicated to a user.

For successful treatment and prevention of lymphedema, an early diagnosis and a suitable appropriate is important. For this early diagnosis, fluorescence imaging is a suitable approach. The distribution of a fluorescent agent that propagates together with the lymphatic fluid and the development of this distribution is analyzed in fluorescence imaging. Before the measurement of the fluorescence initially starts, the fluorescent agent is administered to a tissue of a body part of a patient which shall undergo a medical examination. In a healthy lymphatic system, the fluorescent agent is quickly taken up by the lymphatic system (together with the lymphatic fluid) and rapidly transported in the lymphatic channels. The normal rhythmic contraction of the lymphatics can be observed in time resolved fluorescence images. It can be seen how the fluorescent agent is pushed up the limb while the one way valves in the lymphatic veins prevent a backflow of the fluid.

In case of lymphedema, the obstruction of lymphatic fluid leads to an increase of the pressure inside the lymphatic channels. This causes lymphatic fluid to leak out into subcutaneous tissue. As lymphedema progresses, characteristic backflow patterns can be seen in the fluorescence images.

The fluorescence images are typically captured at two instances. A first image is captured at an early transient phase and at least a second image is captured at a later plateau phase. In this procedure, the patient initially remains still for a few minutes and a certain amount of fluorescent agent is injected subcutaneously. Immediately after injection, in the so-called "transient" phase, the observable fluorescence image shows the lymphatic flow and for example the lymph pump function can be measured. These images are captured while the patient remains still. After that, the patient is allowed to move freely and lymph circulation is evaluated based on observable patterns. These patterns allow a severity staging of lymphedema, for example a severity staging which is mentioned above as defined by WHO. Depending on the degree of movement of the patient, the fluorescent agent can reach the plateau phase at about two hours after injection. Basically, the lymph circulation can be evaluated somewhat between 2 and 72 hours after injection.

The fluorescence image that are analyzed by the CDSS according to embodiments are always fluorescence images that are taken during the later plateau phase, in which the typical lymph backflow patterns can be observed.

Depending on the stage of lymphedema, characteristic backflow patterns that are clinically classified in a number of types, can be seen on the fluorescence images. When the lymphatic system is in healthy condition, a linear pattern can be observed. This linear pattern shows the backflow of the lymphatic fluid in the lymphatic channels or veins. At an early stage of lymphedema, a so-called "splash-back" pattern can be observed. With further progression of lymphedema, a so-called "starburst" pattern occurs. In a severe and high stage of lymphedema, the lymphatic fluid and therewith the fluorescent agent, pours throughout the skin and subcutaneous tissue resulting in a so-called "diffuse" pattern.

Advantageously, the CDSS performs an automatic classification of the lymphedema induced fluorescence patterns and communicates the same to a user. This will significantly help the medical professionals to find the correct diagnosis with respect to lymphedema.

According to an advantageous embodiment, the classification of a lymphedema induced fluorescence pattern is a stage of severity of lymphedema and/or a clinical type of the fluorescence pattern. The stage of severity can for example be a stage according to the definition of WHO. The clinical types have been mentioned above. Possible clinical types that can be identified are for example a linear pattern, a splash pattern, a star dust pattern and a diffuse pattern.

In clinical practice, it is often difficult to discriminate between the different types of fluorescence patterns. It is furthermore sometimes difficult to assign a certain stage of lymphedema to a certain fluorescence image. Advantageously, the CDSS helpfully assists the clinical personnel during this decision process.

According to a further advantageous embodiment, the fluorescence image and a corresponding visible light image are provided through the input interface as input features to the artificial intelligence model. In other words, the fluorescence image and/or a combination of the fluorescence image and the visible light image can be used as input to the artificial intelligence model. The information that can be derived from the visible light image can assist and thereby enhance the process of finding the classification of the fluorescence pattern because clinical finding of lymphedema is accompanied by certain symptoms that are more or less visible on the surface, for example on the outer skin, of the body part.

Within the context of this specification, the term "image" should not be interpreted as being limited to a 2D image. The image can also be a reconstructed 3D image. The image can be captured for example by a stereoscopic imaging unit or it can be calculated from a 2D image plus additional depths information that can be captured for example by a scanner or any other suitable device.

In other words, the terms "fluorescence image" and "visible light image" are also not limited to 2D images. These images can also be 3D images or 2D images comprising additional information, for example depth information. 2D images can be captured using a suitable camera. 3D images can be captured using for example a stereo camera. The 2D or 3D images can also be line scans, which can be captured using an image line scanner. The images can further be LIDAR scans (plus additional color information, which is for example taken from a 2D image) that similar to 3D images also comprise depth information.

The data from the LIDAR scanner can be added to a 2D image as additional information. The combination of the 2D image data and the LIDAR scan data leads to the similar information as a 3D image because the 2D image data can be combined with corresponding depth information.

According to another advantageous embodiment, the CDSS is further enhanced in that the input interface is a direct link to an image capturing and processing device that is configured to measure the fluorescence signal in the tissue of the body part and that is configured to image a surface of the body part, wherein the tissue to which the fluorescent agent has been added forms part of the body part, the image capturing and processing device comprising an image capturing device, which comprises:
an illumination unit configured to illuminate the tissue with excitation light having a wavelength suitable to generate emitted light by excited emission of the fluorescent agent,
a fluorescence imaging unit configured to capture the fluorescence image by spatially resolved measurement of the emitted light so as to provide the fluorescence image,
a visible light imaging unit configured to capture the corresponding visible light image of a section of a surface of the body part, wherein the fluorescence imaging unit and the visible light imaging unit are configured in that a viewing direction and/or a perspective of the fluorescence image and the corresponding visible light image are linked via a known relationship.

Advantageously, the image capturing and processing device can be a part of an endoscope or a laparoscope.

A fluorescence imaging unit and a visible light imaging unit that are configured in that the viewing direction and/or the perspective of the fluorescence image and the visible light image are linked via the known and constant relationship are advantageous in that corresponding images can be captured. The images are linked via the known and constant spatial relationship of the capturing device. This significantly enhances the success rate when applying a stitching algorithm on the images, wherein the stitching results in a larger fluorescence image and a larger visible light image. The larger images are larger in that they show a section of the body part that is greater than the part of the body that can be captured by a single fluorescence and single visible light image, respectively.

According to an embodiment, the CDSS is further enhanced in that a large fluorescence image and a corresponding large visible light image are provided through the input interface as input features to the artificial intelligence model, wherein
the fluorescence imaging unit and the visible light imaging unit are further configured to repeat capturing of the fluorescence image and the visible light image to provide a series of fluorescence images and a series of visible light images,
the image capturing and processing device further comprises a processing device, which comprises:
a stitching unit configured to apply a stitching algorithm on the series of visible light images to generate the large visible light image of the body part, the stitching algorithm determining and applying a set of stitching parameters, wherein the stitching unit is further configured to apply the stitching algorithm on the series of fluorescence images to generate the large fluorescence image, wherein the stitching algorithm applies the set of stitching parameters determined when performing the stitching of the visible light images.

Within the context of this specification, the term "stitching" is not limited to a combination of two or more 2D images. Stitching can also be performed on the basis of other image data, for example on the basis of 3D image data. Further information like for example the depth information that is captured by the LIDAR scanner can be taken into account when executing the stitching algorithm.

Stitching can be performed on the basis of two or more 2D images and results in a larger 2D image. When the stitching algorithm is executed on the basis of a plurality of 3D images, the result is a large 3D image. It is also possible to perform the step of stitching in that a plurality of 2D images plus additional information (for example data from a LIDAR scan or data from a similar device) is processed and the result of this is a large 3D image. In this case, the step of stitching includes the reconstruction of a 3D image from a 2D image data plus additional information. Stitching of images comprises the step of identifying unique special features that correspond to each other and which are visible in the two images that have to be stitched together. This requires that the field of view of the two images that are combined in the stitching process do partly overlap.

In view of this prerequisite for the stitching process, according to an embodiment, the step of repeating the capturing of the fluorescence image and the visible light image to provide the series of fluorescence images and the series of visible light images is executed in that the individual subsequent images of said sequence are captured in that the field of view of the subsequent images are at least slightly overlapping.

The previously mentioned additional information, which can be used for the stitching process, is however not limited to depth information, which is assigned to for example every individual pixel in the 2D image. It is also possible that during the image acquisition of the series of fluorescent images and visible light images, the image capturing device acquires, as further data, i.e. additional information, data about a spatial orientation of the image capturing device. The spatial orientation can be for example an orientation of the image acquisition device in a coordinate system of an examination room. This orientation can be characterized by the three Cartesian coordinates x, y and z together with a viewing direction (for example a vector in the coordinate system of the examination room) and a tilt angle of the image capturing device about the viewing direction (for example an angle of rotation of the image capturing device about the viewing direction). This information can be captured for every single image (or image pair comprising the fluorescence image and the visible light image) of the series of visible light images and fluorescence images. When performing the step of stitching, this additional information indicating the orientation of the image capturing device in space can be used. In particular, the orientation of the image capturing device, which is for example defined in the coordinate system of the examination room, can be recalculated into an orientation of the image capturing device in relation to the body part. The information can be used when performing the 3D reconstruction of the body part during the step of stitching.

Advantageously, the stitching of the series of fluorescence images is performed based on the set of stitching parameters, which have previously been determined when performing the stitching of the visible light images. Fluorescence images typically offer rare special features that are suitable for performance of the stitching process. Because the visible light image and the fluorescence image are linked by the known and constant relationship with respect to viewing direction and/or perspective, the parameters of the stitching algorithm used for the visible light images can also be applied for the stitching of the fluorescence images. This significantly enhances the stitching process and results in a large fluorescence image of better quality.

The fluorescent agent is for example ICG (Indocyanine Green) or Methylene Blue. Within the context of this specification, the term "fluorescent dye" or "dye" (also referred to as "fluorochrome" or "fluorophore") refers to a component of a molecule, which causes the molecule to be fluorescent. The component is a functional group in the molecule that absorbs energy of a specific wavelength and re-emits energy at a different specific wavelength. In various aspects, the fluorescent agent comprises a fluorescence dye, an analogue thereof, a derivative thereof, or a combination of these. Appropriate fluorescent dyes include, but are not limited to, indocyanine green (ICG), fluorescein, methylene blue, isosulfan blue, Patent Blue, cyanine5 (Cy5), cyanine5.5 (Cy5.5), cyanine7 (Cy7), cyanine7.5 (Cy7.5), cypate, silicon rhodamine, 5-ALA, IRDye 700, IRDye 800CW, IRDye 800RS, IRDye 800BK, porphyrin derivatives, Illuminare-1, ALM-488, GCP-002, GCP-003, LUM-015, EMI-137, SGM-101, ASP-1929, AVB-620, OTL-38, VGT-309, BLZ-100, ONM-100, BEVA800.

The stitching algorithm is for example a panorama stitching algorithm, in which the images are analyzed to extract special and distinguishing features. These features are then linked to each other in the multiple images and image transformation (for example shifting, rotation, stretching along one or more axis or a keystone correction) is performed. The location of the linked features is used to determine the image transformation parameters, which are also referred to as the stitching parameters. Subsequent to image orientation, the images are merged and thereby "stitched" together. This transformation can be executed in the same way on both, the visible light images and the fluorescence images. Finally, the two images are output. This output can include the step of displaying the images for example on a screen. For example the two images are shown side-by-side or in an overlay image.

Often, the function of the lymphatic system is affected in a limb of the body. However, the CDSS is not limited to the inspection of a limb. The system can be applied for examination of a body part in general, which can be a limb of a patient but also the corpus, the head, the neck, the back or any other part of the body. It is also possible that the body part is an organ. The CDSS can be applied during open surgery. The same applies to a situation in which the surgery is minimally invasive surgery, which is performed using an endoscope or laparoscope.

Within the context of this specification, a "visible light image" is an image of the real world situation. It reproduces an image impression similar to what can be seen by the human eye. Unlike the human eye, the visible light image can be a color image, a greyscale image or even a false color scale plot. The visible light image shows the surface of the body part comprising the tissue to which the fluorescent agent has been administered. If the tissue is arranged at the surface of the body part, the imaging of the surface of the body part includes imaging of the surface of the tissue.

According to another advantageous embodiment, the viewing direction and the perspective of the fluorescence image and the visible light image are identical and, in particular, the fluorescence image and the visible light image are captured through one and the same, in particular through one single, objective lens. The objective lens is similar to a camera lens and comprises one or more lenses.

Advantageously, the fluorescence image and the visible light image can be captured by an imaging device, which comprises a prism assembly and a plurality of image sensors assigned thereto. Fluorescent light and visible light enter the prism assembly as a common light bundle and through one and the same entrance surface of the prism assembly. The prism structure comprises filters for separating the visible wavelength range from the infrared wavelength range, at which the exited emission of the fluorescent agent typically takes place. The different wavelength bands, i.e. the visible light (also abbreviated Vis) and the infrared light (also abbreviated IR), are directed to different sensors. The capturing of the visible light image and the fluorescence image through one single objective lens allows a perfect alignment of the viewing direction and perspective of the two images. In particular, the viewing direction and the perspective of the visible light image and the fluorescence image are identical.

In an advantageous embodiment, capturing of the fluorescence image and capturing of the visible light image are performed simultaneously in absence of time-switching between a signal of the fluorescence image and a signal of the visible light image.

Advantageously, the method dispenses with time-switching of signals. This is advantageous, because the infrared image, which is the fluorescence image, and the visible light image are captured exactly at the same time using separate image sensors. Hence, the images can also be captured with a high frame repeat rate of 60 fps or even higher. High frame rates can typically not be achieved when time-switching is applied. Furthermore, when the fluorescence image and the visible light image are captured on individual sensors, the sensors can be arranged exactly in focus. This enhances the sharpness of the images. Furthermore, the settings of the sensors can be adjusted to the individual requirements for image acquisition of the visible light image and fluorescence image. This pertains for example to an adjustment of the sensor gain, the noise reduction, the exposure time, etc..

According to yet another advantageous embodiment, the steps of capturing the fluorescence image, illuminating the tissue with excitation light and simultaneously capturing the visible light image are performed by a single image capturing device. When illumination and image acquisition are integrated in one device, the overall process of measurement of the fluorescence signal and simultaneous acquisition of visible images can be enhanced.

Furthermore, according to another advantageous embodiment, the CDSS is further enhanced in that the image capturing device comprises a dichroic prism assembly configured to receive fluorescent light and visible light through an entrance face, the prism assembly comprising: a first prism, a second prism, a first compensator prism located between the first prism and the second prism,
a further dichroic prism assembly for splitting the visible light in three light components, and a second compensator prism located between the second prism and the further dichroic prism assembly,
wherein the first prism and the second prism each have a cross section with at least five corners, each corner having an inside angle of at least 90 degrees, wherein the corners of the first prism and the second prism each have a respective entrance face and a respective exit face, and are each designed so that an incoming beam which enters the entrance face of the respective prism in a direction parallel to a normal of said entrance face is reflected twice inside the respective prism and exits the respective prism through its exit face parallel to a normal of said exit face,
wherein the normal of the entrance face and the normal of the exit face of the respective prism are perpendicular to each other; and
wherein, when light enters the first prism through the entrance face, the light is partially reflected towards the exit face of the first prism thereby traveling a first path length from the entrance face of the first prism to the exit face of the first prism, and the light partially enters the second prism via the first compensator prism and is partially reflected towards the exit face of the second prism, thereby traveling a second path length from the entrance face of the first prism to the exit face of the second prism,
wherein the first prism is larger than the second prism so that the first and the second path lengths are the same.

Advantageously, the above-referred five prism assembly allows to capture two fluorescence imaging wavelengths and the three colors for visible light imaging, for example red, blue and green. The five prism assembly is advantageous in that the optical paths of the light traveling from the entrance surface to a respective one of the sensors have identical length. Hence, all sensors are in focus and furthermore, there is no timing gap between the signals of the sensors. Advantageously, the device does not require time-switching of the received signals.

According to still another aspect, the image capturing device, defines a first, a second, and a third optical path for directing fluorescence light and visible light to a first, a second, and a third sensor, respectively, the image capturing device further comprises a dichroic prism assembly, configured to receive the fluorescent light and the visible light through an entrance face, the dichroic prism assembly comprising: a first prism, a second prism and a third prism, each prism having a respective first, second, and third exit face, wherein: the first exit face is provided with the first sensor, the second exit face is provided with the second sensor, and the third exit face is provided with the third sensor, wherein in particular the first optical path is provided with a first filter, the second optical path is provided with a second filter, and the third optical path is provided with a third filter, wherein
the first, second, and third filters, in any order, are a green filter, an infrared filter, and a red/blue patterned filter comprising red and blue filters in alternating pattern so that half of the light received by the red/blue patterned filter goes through a blue filter and half of the light received by the red/blue patterned filter goes through a red filter.

Furthermore, according to another aspect, the first, second, and third filters, in any order, are a red/green/blue patterned filter (RGB filter), a first infrared filter, and a second infrared filter, wherein in particular, the first and second infrared filter have different transmission wavelength.

In other words, the first and second infrared filter are for filtering IR-light in different IR wavelength intervals, for example in a first IR-band in which typical fluorescent dyes emit a first fluorescence peak and in a second IR-band in which a typical fluorescent dye emits a second fluorescence peak. Typically, the second IR-band is located at higher wavelength compared to the first IR-band. The first and second infrared filter can also be adjusted to emission bands of different fluorescent agents. Hence, the emission of for example a first fluorescent agent passes the first filter (and is in particular blocked by the second filter) and can be detected on the corresponding first sensor and the emission of the second fluorescent agent passes the second filter (and is in particular blocked by the first filter) and can be detected on the corresponding second sensor. For example, the first filter can be configured to measure the fluorescence emission of methylene blue and the second filter can be configured to measure the fluorescence emission of ICG.

According to an advantageous aspect of the invention, the CDSS is further enhanced in that the illumination unit, the fluorescence imaging unit and the visible light imaging unit are arranged in a single image capturing device, which further comprises a measurement unit configured to measure a distance between the surface of the body part, which is captured in the visible light image. Furthermore, the CDSS can be enhanced in that the image capturing device is further configured to output a signal, which is indicative of the measured distance. Measurements at different distances can be performed to optimize the illumination and image capturing to find the best image acquisition conditions. The distance of this best fit can then be stored in the imaging system as a target distance for following measurements.

For example, a visual signal and/or an audio signal can be output by the image capturing device. This signal can guide the operator when handling the image capturing device such that image acquisition is performed at an at least approximately constant distance to the surface of the body part. The integration of the distance sensor in the image capturing device and the user supporting output (visual or optical signal) enable the operator to capture images with more homogeneous illumination. This enhances the quality of the measurement of the fluorescence signal.

For determination of the best fit distance, the image capturing device can repeatedly capture the fluorescence image and the visible light image of the same section of the surface of the body part while measuring the distance. A plurality of sets of fluorescence and visible light images can be captured at different distances. Subsequently, an analysis of the sets of images in view of imaging quality can be performed and a best matching distance resulting in the highest quality of images can be determined. In view of this, the output signal, which can be an audio signal or an optical signal, can also be indicative of a deviation of the measured distance from the best matching distance. Hence, the operator is directly informed whether or not the optimum image capturing conditions in particular with respect to illumination are applied during the measurement.

According to still another advantageous embodiment of the invention, the CDSS is further enhanced in that the input interface is furthermore a direct link to an electronic patient record, wherein patient related data, in particular data relative to age, gender, height, weight, BMI (Body Mass Index), fat mass, muscle mass, daily exercise mass, presence or absence of work, skin color, medication status, presence or absence of vascular disease, in particular varicose veins or venous edema, presence or absence of disease, in particular dialysis or diabetes, amount of albumin in blood, kidney function, liver function, heart function, Hemoglobin (Hb) concentration in the blood, blood estimate, lipid metabolism, blood glucose concentration in the blood, urea or nitrogen (BUN in the blood, the amount of UN), ABI (ankle/humeral index) value; lymphatic function measurement data at the same location (40 A) before the occurrence of lymphaedema, endocrine information, hormone level of the patient, are provided through the input interface as further input features to the artificial intelligence model. By further taking into account this additional input information, the output, namely the classification of the lymphedema induced for fluorescence pattern can be enhanced. For example, the input interface can comprise a piece of hardware that allows the user to manually enter the data, for example data relative to age, gender, height, weight, BMI (Body Mass Index), fat mass, muscle mass, daily exercise mass, presence or absence of work, skin color, medication status, presence or absence of vascular disease, in particular varicose veins or venous edema, presence or absence of disease, in particular dialysis or diabetes, amount of albumin in blood, kidney function, liver function, heart function, Hemoglobin (Hb) concentration in the blood, blood estimate, lipid metabolism, blood glucose concentration in the blood, urea or nitrogen (BUN in the blood, the amount of UN), ABI (ankle/humeral index) value; lymphatic function measurement data at the same location (40 A) before the occurrence of lymphaedema, endocrine information, hormone level of the patient. The download of this information from an electronic patient record, if available, will speed up this process.

As mentioned before, the input features of the AI model can be either 2D images or 3D images, wherein fluorescence images can be a combination of visible light images and fluorescence images. Furthermore, it is possible to use more than one fluorescent agent. For example fluorescent agents like ICG (Indocyanine Green) and Methylene Blue can be used. The analyzed images can be single dye images, combined fluorescent images, i.e. more than one dye, which can further be combined with visible light images. It can be stitched images or reconstructed images. It is also possible to process overlay images comprising for example a visible light image and a fluorescence image.

Based on this information, the AI model can perform image segmentation using algorithms like watershed, thresholding, clustering, histogram or the like. It is also possible to define a user defined region of interest and/or to segment lymphatic details in the images.

The software module for performing the interference operation based on the AI model can be a pre-trained AI model for classifying fluorescence patterns. The AI can also comprise a plurality of pre-trained AI models. The user can than choose between the different AI models or systems and select the one that suits best his or her needs.

According to still another advantageous embodiment, the input interface can comprise a user interface for correcting the automatically generated classification of the fluorescence pattern. This in particular applies to the automatic generation of the stage of severity and clinical type of fluorescence pattern. The corrected classification can be stored or assigned in relation to the fluorescence images and patient related data in addition to the automatic result relative to the classification that is the output by the AI model. It is also possible to overwrite the AI predicted data.

According to still another advantageous embodiment, the AI model can be customized by user-specific training. The user can select a training mode and input own data which means own fluorescence images as a ground truth. The user-trained AI model will then be stored in addition to the pre-trained models. The user can select which AI model to use. The user-trained AI model can be particularly advantageous if for example an own staging system that deviates from the common staging system of lymphedema, for example from the WHO definition, shall be used.

The object is further solved by a computer implemented method of determining a classification of a lymphedema induced fluorescence pattern using a computer based clinical decision support system (CDSS), the classification being based on a fluorescence image that is determined by measuring a fluorescence signal in a tissue of a body part, to which a fluorescent agent has been added, the method comprising:
receiving the fluorescence image, which is specific to a patient, through an input interface as an input feature of an artificial intelligence (AI) model,
performing an inference operation by a processor, in which the fluorescence image is applied to the AI model to generate the classification of a lymphedema induced fluorescence pattern, and
communicating the classification of a lymphedema induced fluorescence pattern to a user through a user interface (III).

Same or similar advantages that have been mentioned with respect to the CDSS also apply to the computer implemented method in a same or similar way and shall be therefore not repeated.

Furthermore, the method can be advantageously enhanced in that the classification of a lymphedema induced fluorescence pattern is a stage of severity of lymphedema and/or a clinical type of the fluorescence pattern.

Furthermore, the method is advantageously enhanced in that the fluorescence image and a corresponding visible light image are provided through the input interface as input features to the artificial intelligence (Al) model.

According to still another advantageous embodiment, the method is enhanced in that the input interface receives the fluorescence image and the corresponding visible light image through a direct link to an image capturing and processing device that is configured to measure the fluorescence signal in the tissue of the body part and that is configured to image a surface of the body part, wherein the tissue to which the fluorescent agent has been added forms part of the body part, and wherein the image capturing and processing device comprises an image capturing device, which comprises an illumination unit, a fluorescence imaging unit and a visible light imaging unit, the method further comprising the steps of:
illuminating of the tissue by the illumination unit with excitation light having a wavelength suitable to generate emitted light by excited emission of the fluorescent agent,
capturing of the fluorescence image by the fluorescence imaging unit by spatially resolved measurement of the emitted light so as to provide the fluorescence image,
capturing of the visible light image by the visible light imaging unit by capturing the corresponding visible light image of a section of a surface of the body part, wherein the fluorescence imaging unit and the visible light imaging unit are configured in that a viewing direction and/or a perspective of the fluorescence image and the corresponding visible light image are linked via a known relationship.

Furthermore, this method can be advantageously enhanced in that a large fluorescence image and a corresponding large visible light image are provided through the input interface as input features to the artificial intelligence (AI) model, and wherein
the fluorescence imaging unit and the visible light imaging unit repeat capturing of the fluorescence image and the visible light image to provide a series of fluorescence images and a series of visible light images, wherein
the image capturing and processing device further comprises a processing device, which comprises a stitching unit and the method further comprises the steps of:
   applying a stitching algorithm on the series of visible light images by the stitching unit to generate the large visible light image of the body part, the stitching algorithm determining and applying a set of stitching parameters,
   further applying the stitching algorithm on the series of fluorescence images by the stitching unit to generate the large fluorescence image, wherein the stitching algorithm applies the set of stitching parameters determined when performing the stitching of the visible light images.

It is furthermore advantageous if the viewing direction and the perspective of the fluorescence images and the visible light image are identical. In particular, the fluorescence image and the visible light image are captured through one of the same objective lens.

Furthermore, the method can be enhanced in that the capturing of the fluorescence image and the capturing of the visible light image are performed simultaneously, in particular in absence of time switching between a signal of the fluorescence image and a signal for the visible light image.

Furthermore, according to an advantageous embodiment, the steps of capturing the fluorescence image, illuminating the tissue with excitation light and simultaneously capturing the visible light image are performed by a single image capturing device.

It is also advantageous, when according to another embodiment, the method further comprises steps of measuring a distance between a surface of a body part, which is captured in a visible light image and the capturing device. Furthermore, a signal can be output by the imaging device which is indicative of the measured distance. The method can furthermore comprise the steps of repeatedly capturing the fluorescence image and the visible light image of the same section of the surface of the body part while measuring the distance. The plurality of sets of fluorescence and visible light images can be captured at different distances. The sets of images can be analyzed in view of imaging quality and a best matching distance can be determined resulting the highest quality of images. Hence, the output signal can be indicative of deviation of the measured distance from the best matching distance.

According to still another advantageous embodiment, the method of determining a classification of a lymphedema induced fluorescence pattern can be further enhanced in that the measurement of the fluorescence signal is performed on a tissue, to which at least a first and a second fluorescent agent has been added, wherein the step of capturing the fluorescence image comprises:
capturing a first fluorescence image in a first wavelength range, which is generated by illuminating the tissue with first excitation light having a first wavelength suitable to generate emitted light by a first excited emission of the first fluorescent agent, and
capturing a second fluorescence image in a second wavelength range, which is generated by illuminating the tissue with second excitation light having a second wavelength suitable to generate emitted light by a second excited emission of the second fluorescent agent, and wherein
the first and the second fluorescence are provided through the input interface as input features to the artificial intelligence (Al) model, wherein
the input interface receives the first and the second fluorescence image as an input features of the artificial intelligence (AI) model, and
the processor performs the inference operation by applying the first and the second fluorescence image to the AI model to generate the classification of the lymphedema induced fluorescence pattern.

Furthermore, the method can be enhanced in that patient related data, in particular data relative to sex, age or BMI of the patient, is provided through the input interface as further input features to the artificial intelligence (Al) model, via a direct link to an electronic patient record.

The object is further solved by a method of diagnosing lymphedema, comprising the steps of:
administering a fluorescent agent to a body part of a patient,
determining a classification of a lymphedema induced fluorescence pattern using a computer based clinical decision support system (CDSS), wherein the classification is based on a fluorescence image that is determined by measuring a fluorescence signal in a tissue of the body part, to which a fluorescent agent has been added, the method further comprising:
   receiving the fluorescence image, which is specific to a patient, through an input interface as an input feature of an artificial intelligence (AI) model,
   performing an inference operation by a processor, in which the fluorescence image is applied to the AI model to generate the classification of a lymphedema induced fluorescence pattern, and
   deriving a diagnostic result from the classification of a lymphedema induced fluorescence pattern, in particular a diagnostic result relative to a stage of lymphedema,
   communicating the classification of a lymphedema induced fluorescence pattern and the diagnostic result to a user through a user interface (III).

The method of diagnosing lymphedema can advantageously be performed with a higher level of precision and reliability. The method does advantageously not rely on decisions of medical professionals that interpret fluorescence patterns. Such decisions inevitably suffer in quality from personal experience of the medical person. The method is more objective and thereby precise.

The object is further solved by a method of long-term therapy of lymphedema, comprising the steps of:
diagnosing a severity of lymphedema by performing the method of diagnosing lymphedema according to one or more of the above-mentioned embodiments on a patient,
performing a therapy on the patient, the therapy being customized to the diagnostic result relative to the severity of lymphedema,
repeating the steps of diagnosing the severity of lymphedema and performing of the therapy on the patient, wherein in each iteration, the therapy is adjusted to the detected stage of lymphedema, in particular to the detected stage of lymphedema.

The method of long term therapy according to aspects of the invention is in particular useful because the diagnosis of lymphedema provides - in contrast to traditional methods - objective results with respect to the severity of the disease. The success of a long term therapy can be analyzed from an objective point of view.

The CDSS applies an artificial intelligence model. Machine learning (ML) is a field of artificial intelligence. Machine learning algorithms build a model based on sample data, known as training data, in order to make predictions or decisions without being explicitly programmed to do so.

There are two common modes for machine learning (ML): supervised ML and unsupervised ML. Supervised ML uses prior knowledge (e.g., examples that correlate inputs to outputs or outcomes) to learn the relationships between the inputs and the outputs. The goal of supervised ML is to learn a function that, given some training data, best approximates the relationship between the training inputs and outputs so that the ML model can implement the same relationships when given inputs to generate the corresponding outputs. Unsupervised ML is the training of an ML algorithm using information that is neither classified nor labeled, and allowing the algorithm to act on that information without guidance. Unsupervised ML is useful in exploratory analysis because it can automatically identify structure in data.

Common tasks for supervised ML are classification problems and regression problems. Classification problems, also referred to as categorization problems, aim at classifying items into one of several category values (for example, is this object an apple or an orange?). Regression algorithms aim at quantifying some items (for example, by providing a score to the value of some input). Some examples of commonly used supervised-ML algorithms are Logistic Regression (LR), Naive-Bayes, Random Forest (RF), neural networks (NN), deep neural networks (DNN), matrix factorization, and Support Vector Machines (SVM).

Some common tasks for unsupervised ML include clustering, representation learning, and density estimation. Some examples of commonly used unsupervised-ML algorithms are K-means clustering, principal component analysis, and autoencoders.

Another type of ML is federated learning (also known as collaborative learning) that trains an algorithm across multiple decentralized devices holding local data, without exchanging the data. This approach stands in contrast to traditional centralized machine-learning techniques where all the local datasets are uploaded to one server, as well as to more classical decentralized approaches which often assume that local data samples are identically distributed. Federated learning enables multiple actors to build a common, robust machine learning model without sharing data, thus allowing to address critical issues such as data privacy, data security, data access rights and access to heterogeneous data.

In some examples, the AI model may be trained continuously or periodically prior to performance of the inference operation by the processor. Then, during the inference operation, the patient specific input features provided to the AI model may be propagated from an input layer, through one or more hidden layers, and ultimately to an output layer that corresponds to the classification of the lymphedema induced fluorescence pattern. For example, the stage of severity or the level of lymphedema and/or a clinical type of the fluorescence pattern, for example one of linear pattern, splash pattern, stardust pattern or diffuse pattern.

During and/or subsequent to the inference operation, the classification of the lymphedema induced fluorescence pattern may be communicated to the user via the user interface (UI). For example, the fluorescence image or the overlay image can be displayed on a display together with the information on the lymphedema induced fluorescence pattern. This can be a report indicating for example the level of lymphedema or the clinical type of fluorescence pattern that correspond to the AI generated confidence level. The report can also include the suggested diagnosis and/or treatment option.

Further characteristics of the invention will become apparent from the description of the embodiments according to the invention together with the claims and the included drawings. Embodiments according to the invention can fulfill individual characteristics or a combination of several characteristics.

The invention is described below, without restricting the general intent of the invention, based on exemplary embodiments, wherein reference is made expressly to the drawings with regard to the disclosure of all details according to the invention that are not explained in greater detail in the text.
- Fig. 1: illustrates a schematic view on an image capturing and processing device forming a part of a CDSS,
- Fig. 2: illustrates a schematic view on an image capturing device and a processing unit of the imaging capturing and processing device,
- Fig. 3a): illustrates an example of a visible light image and
- Fig. 3b): illustrates the corresponding fluorescence image,
- Fig. 4: illustrates a large overlay image, which is in part generated from the visible light and fluorescence images shown in Figs. 3a) and 3b),
- Fig. 5: illustrates an internal prism assembly of the image capturing device,
- Fig. 6: schematically illustrates an endoscope or laparoscope including the image capturing device,
- Fig. 7: illustrates a flowchart of a stitching algorithm.
- Fig. 8.: schematically illustrates a computer based clinical decision support system (CDSS),
- Fig. 9 A-D: illustrate examples of fluorescence patterns,
- Fig. 10: schematically illustrates another internal prism assembly of the image capturing device.

In the drawings, the same or similar types of elements or respectively corresponding parts are provided with the same reference numbers in order to prevent the item from needing to be reintroduced.

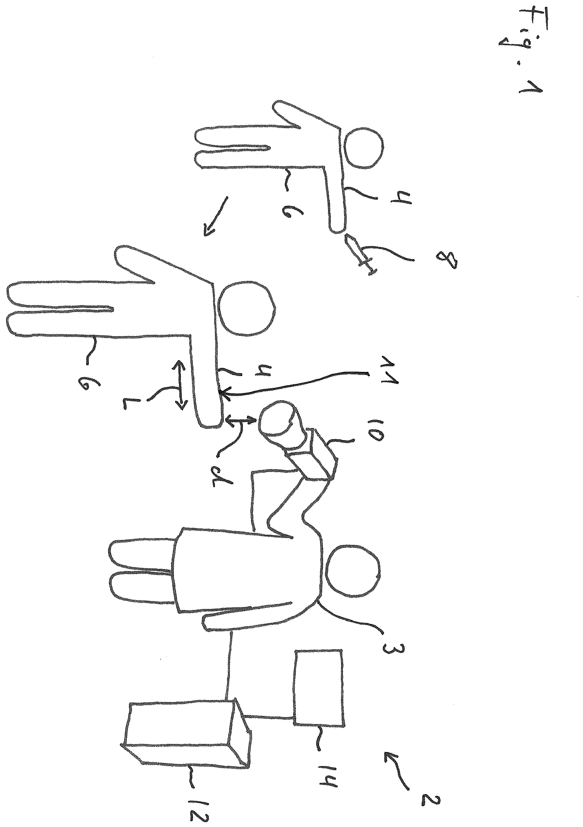

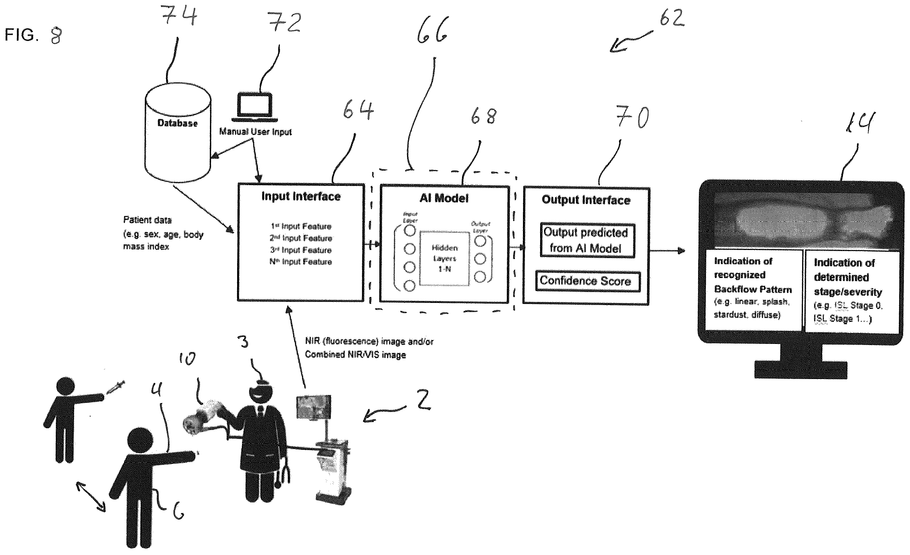

Fig. 1 illustrates an image capturing and processing device 2, which can serve as an input interface to a computer-based clinical decision support system (CDSS). The CDSS will be explained when making reference to Fig. 8.. Initially, the focus is on the data acquisition by the image capturing and processing device 2.

The image capturing and processing device 2 is configured to measure a fluorescence signal in a tissue of a body part 4 of a patient 6. By way of an example only, the body part 4 of the patient 6, which is inspected, is the arm. The measurement of the fluorescence signal can also be performed on other body parts 4 of the patient 6, for example the leg, a part of the head, neck, back or any other part of the body. The measurement can also be performed during open surgery. In this application scenario, the body part 4 can be for example an inner organ of the patient 6. The measurement of the fluorescent signal can also be performed during minimally invasive surgery. For this application scenario, the image capturing and processing devices 2 is at least partly integrated for example in an endoscope or laparoscope. For example, the endoscope or laparoscope comprises the image capturing device 10.

Before the measurement initially starts, a fluorescent agent 8 is administered, i.e. injected, in the tissue of the patient's body part 4. The capturing of the fluorescence signal by the image capturing and processing device 2 in the tissue of the body part 4 excludes the step of administering the fluorescent agent 8.

The fluorescent agent 8 is for example ICG. ICG (Indocyanine Green) is a green colored medical dye that is used for over 40 years. ICG emits fluorescent light when exited with near infrared light having a wavelength between 600 nm and 800 nm. The emitted fluorescence light is between 750 nm and 950 nm. It is also possible that the fluorescent agent 8 comprises two different medical dyes. For example, the fluorescent agent 8 can be a mixture of Methylene Blue and ICG.

Subsequent to the administration of the fluorescent agent 8, as it is indicated by an arrow in Fig. 1, the patient's body part 4 can be inspected using an image capturing device 10, which forms part of the image capturing and processing device 2.

During fluorescence imaging of lymphedema, images are typically captured at two instances. The first image is captured at an early transient phase, when the fluorescent agent is quickly taken up by the lymphatic system, and at a second instance at a later plateau phase. All fluorescence images that are processed by the CDSS are captured during this later plateau phase.

The image capturing device 10 is configured to image a surface 11 of the body part 4 and to detect the fluorescence signal, which results from illumination of the fluorescent agent 8 with excitation light. When the image capturing device 10 is applied in surgery, the surface 11 of the body part 4 is a surface of for example an inner organ. In this case, the surface 11 of the body part 4 is identical to the surface of the tissue, to which the fluorescent agent 8 has been administered. For emission of light having a suitable excitation wavelength, the image capturing device 10 comprises an illumination unit 16 (not shown in Fig. 1).

The captured images are communicated to a processing device 12, which can implement the CDSS. The is CDSS is configured to output a classification of a lymphedema induced fluorescence pattern, based on a fluorescence image that is captured by the image capturing device 10. According to this embodiment, the image capturing a device of the image capturing and processing device into serves as an input interface to an artificial intelligence model.

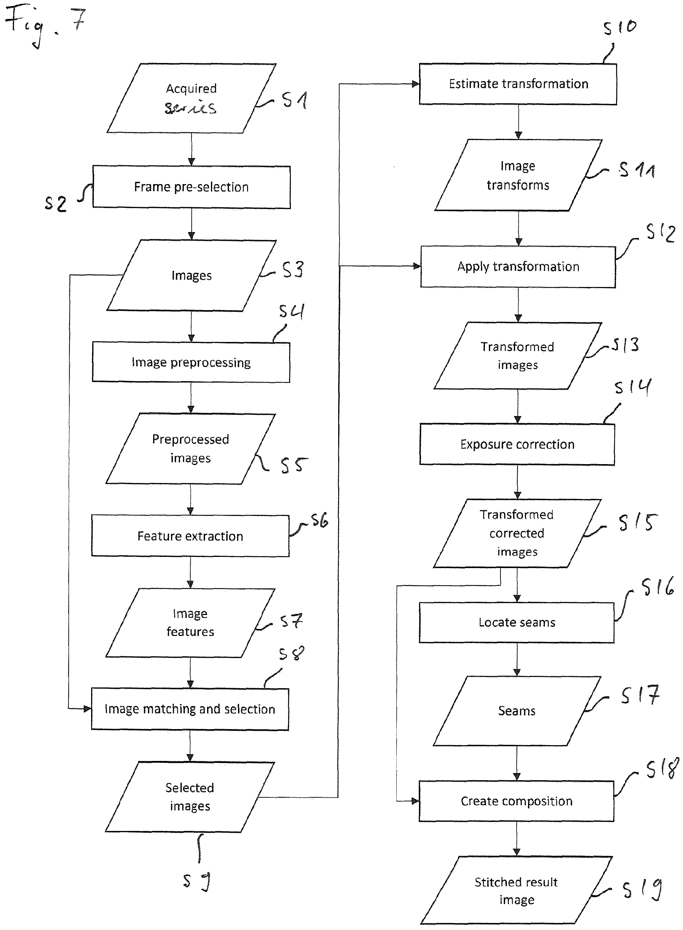

The input interface is not necessarily provided by the image capturing and processing device 2. This will be explained later when making reference to Fig. 8. In the embodiment, which is shown in Fig. 1, the input interface is provided by the image capturing device 10, as previously mentioned. It provides a fluorescence image, which is specific to the patient is 6, to an artificial intelligence (AI) model, which is implemented in the processing device 12. A processor of the processing device 12 performs an interference operation in which the fluorescence image is applied to the AI model to generate the classification of the lymphedema induced fluorescence pattern. Typical lymphedema induced fluorescence patterns are shown in Fig. 7, which will be explained in more detail further below.

The classification of lymphedema induced fluorescence patterns is communicated to a user, in given embodiment to a physician 3. The output is performed via a user interface, which can be the display 14. Together with the output of the AI model, i.e. the classification of the lymphedema induced fluorescence pattern, the fluorescence image and a visible light image can be output on the display 14. The image capturing device 10 can be handled by the physician 3.

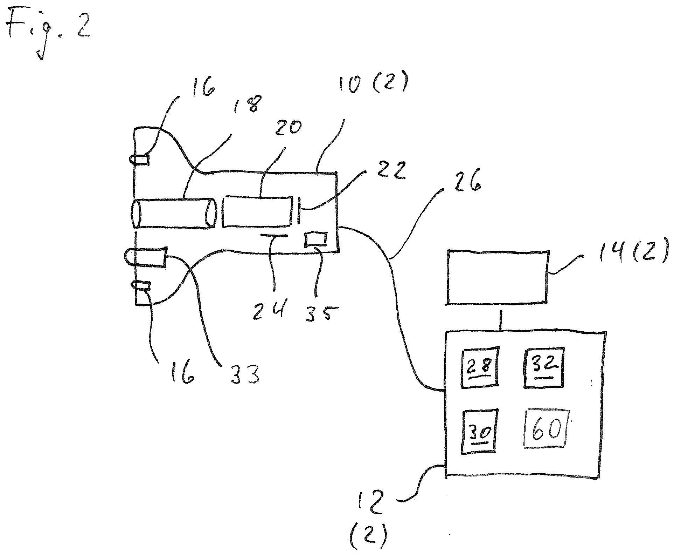

Fig. 2 illustrates the image capturing device 10 and the processing unit 12 of the image capturing and processing device 2 in more detail. The image capturing device 10 comprises an illumination unit 16 which is configured to illuminate the tissue with excitation light having a wavelength suitable to generate fluorescent light by exciting emission of the fluorescent agent 8. For example, a plurality of LEDs is provided in the illumination unit 16.

The image capturing device 10 further comprises an objective lens 18 through which visible light and a fluorescence light are captured. Light is guided through the objective lens 18 to a prism assembly 20. The prism assembly 20 is configured to separate fluorescent light, which is in particular in a wavelength range between 750 nm and 950 nm, from visible light that results in the visible light image. The fluorescent light is directed on a fluorescence imaging unit 22, which is for example a CCD or CMOS sensor plus additional wavelength filters and electronics, if necessary. The fluorescence imaging unit 22 is configured to capture a fluorescence image by spatially resolved measurement of the emitted light, i.e. the excited emission of the fluorescent agent 8, so as to provide the fluorescence image. Furthermore, there is a visible light imaging unit 24, which can be another CCD or CMOS sensor plus an additional different wavelength filter and electronics, if necessary. The prism assembly 20 is configured to direct visible light on the visible light imaging unit 24 so as to allow the unit to capture the visible light image of a section of a surface 11 of the patient's body part 4. Similarly, the prism assembly 20 is configured to direct fluorescent light on the fluorescence imaging unit 22. The prism assembly 20, the fluorescence imaging unit 22 and the visible light imaging unit 24 will be explained in detail further below.

The image capturing device 10 can also be a scanning unit, for example an image line scanning unit or a LIDAR scanning unit. The image capturing device 10 can also be 3D camera, which is suitable to capture a pair of stereoscopic images from which a 3D image including depth information can be calculated. Naturally, the image capturing device 10 can be a combination of these devices.

The image data is communicated from the image capturing device 10 to the processing device 12 via a suitable data link 26, which can be a wireless datalink or a wired data link, for example a data cable.

The image capturing device 10 is configured in that the fluorescence imaging unit 22 and the visible light imaging unit 24 are operated to simultaneously capture the visible light image and the fluorescence image. In particular, the image capturing device 10 does not perform time switching between the signal of the fluorescence image and the signal of the visible light image. In other words, the sensors of the fluorescence imaging unit 22 and the visible light imaging unit 24 are exclusively used for capturing images in the respective wavelength range, which means that the sensors of the imaging units 22, 24 are used for either capturing a fluorescence image in the IR spectrum or for capturing a visible light image in the visible spectrum. The sensors 22, 24 are not used for capturing images in both wavelength ranges. This results in significant advantages. For example, the sensors can be exactly positioned in focus, which is not possible when an image sensor is used for both purposes, i.e. to capture visible light and infrared light, because the focus point for these different wavelengths typically differ in position. Furthermore, the sensor parameters can be adjusted individually, for example with respect to a required exposure time or sensor gain. Individual settings are advantageous because IR signals are typically lower than visible light signals.

The fluorescence imaging unit 22 and the visible light imaging unit 24 have a fixed spatial relationship to each other. This is because the units are arranged in one single mounting structure or frame of the image capturing device 10. Furthermore, the fluorescence imaging unit 22 and the visible light imaging unit 24 use the same objective lens 18 and prism assembly 20 for imaging of the fluorescence image and the visible light image, respectively. Due to these measures, the fluorescence imaging unit 22 and the visible light imaging 24 are configured in that a viewing direction and a perspective of the fluorescence image and the visible light image are linked via a known and constant relationship. In the given embodiment, the viewing direction of the two images are identical and both units 22, 24 image via the same objective lens 18.

The image capturing device 10 can be configured to operate the fluorescence imaging unit 22 and the visible light imaging unit 24 to repeat the capturing of the fluorescence image and the visible light image so as to provide a series of fluorescence images and a series of visible light images. This operation can be performed by the processing device 12 operating the image sensor of the fluorescence imaging unit 22 and the image sensor of visible light imaging unit 24. The series of images is typically captured while an operator or physician 3 (see Fig. 1) moves the image capturing device 10 along a longitudinal direction L of the body part 4 of the patient 6. This movement can be performed in that subsequent images of the series of images comprise overlapping parts. In other words, details which are shown in a first image of the series of images are also shown in a subsequent second image of the series. This is important for the subsequent optional stitching process. To safeguard that corresponding features can be found in subsequent images, the frequency of image acquisition can be set to a sufficiently high value. The capturing of the images can be manually initiated by for example the physician 3 or the capturing of images can be controlled by the image capturing device 10 in that the described prerequisite is fulfilled.

The image capturing device 10 can be further configured to acquire a position and orientation of the image capturing device 10 during this movement. For example, a position and orientation of the image capturing device 10 in a reference system of the examination room or in a reference system of the patient 6 can be determined for each image or image pair that is captured. This information can be stored and communicated to together with the image or image pair comprising the visible image and the fluorescence image. This information can be useful for the subsequent reconstruction of images so as to generate a 3D image from the series of 2D images.

Once the two series of images (i.e. a first series of visible light images and a second series of fluorescence images) or the series of image pairs (each image pair comprising a fluorescence image and a visible light image) are captured by the capturing device 10 and received in the processing device 12, the series of visible light images can be processed by a stitching unit 28 (see Fig. 2). The stitching unit 28 is configured to apply a stitching algorithm on the series of visible light images to generate a large visible light image of the body part 4. The large image is "larger" in that it shows a greater section of the body part 4 of the patient 6 in comparison to a single image.

Within the context of this specification, the term "stitching" shall not be understood in that the process of stitching is limited to a combination of two or more 2D images. Stitching can also be performed on the basis of 3D images, wherein the result of this process is a larger 3D image. The process of stitching can also be performed on the basis of 2D images plus additional information on the direction of view, from which the 2D images have been captured. Further information on the position of the image capturing device 10 can also be taken into account. As mentioned before, on the basis of these data sets, a larger 3D image can be generated, i.e. stitched together from a series of 2D images plus information on the position and orientation of the image capturing device 10. It is also possible to combine 3D scanning data, for example from a LIDAR sensor, with 2D image information. Also in this case, the result of the stitching process is a larger 3D image. The term stitching in particular encompasses the process of reconstructing a 3D image from a dataset.

Irrespective of the particular type of data that is processed during the stitching, the stitching algorithm starts with stitching of the visible light images. The stitching algorithm generates and applies a set of stitching parameters when preforming the stitching operation. The detailed operation of the stitching unit 28 will be described further below. The stitching unit 28 is configured to apply the stitching algorithm not only on the series of visible light images but also on the series of fluorescence images so as to generate a large fluorescence image. Also in this case, the process of stitching is not limited to the combination of two or more 2D images. It is also possible to generate a 3D fluorescence image in a similar way as it is described above for the visible light images.

The stitching algorithm, which is applied for stitching of the fluorescence images is the same algorithm which is used for stitching of the visible light images. Furthermore, the stitching of the fluorescence images is performed using the same set of stitching parameters which was determined when performing the stitching of the visible light images. This is possible, because there is a fixed relationship between the viewing direction and perspective of the visible light images and the fluorescence images. Naturally, if the viewing direction and perspective of the visible light images and the fluorescence images are not identical, a fixed offset or a shift in the stitching parameters has to be applied. This takes into account the known and fixed spatial relationship between the IR and Vis image sensors and the corresponding optics.

Subsequent to the stitching, the large visible light image and the large fluorescence image are available and can serve as input to the AI model. Furthermore, the large visible light image and the large fluorescence image can be output. For example, the images are displayed side-by-side on the display 14 together with the result of the AI model, i.e. the classification of the lymphedema induced fluorescence pattern.

Unlike traditional inspection systems, the display 14 can show a visible light image and a fluorescence image that correspond to each other together with the result of the AI model. Details that can be seen on the fluorescence image, for example a high fluorescence intensity that indicates an accumulation of lymphatic fluid or a characteristic pattern of lymphatic backflow, can be found in the patient's body part 4 exactly on the corresponding position, which is shown in the visible light image. This enables the physician 3 to exactly spot areas in which for example an accumulation of lymphatic fluid is found. This is very valuable information for example for a tailored and specific therapy of the patient 6.

It is also possible that the visible light image and the fluorescence image, in particular the large visible light image and the large fluorescence image are superimposed so as to provide an overlay image, in particular in a large overlay image, of the body part 4. This process can be performed by a superimposing unit 30 of the processing device 12. The overlay image can also be output via the display 14.

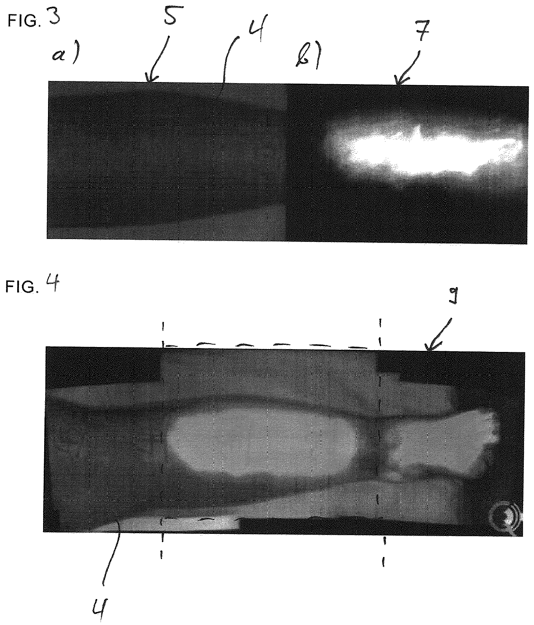

Fig. 3a) shows an example of a visible light image 5, in which a section of a surface 11 of the body part 4 of the patient 6 is visible. By way of an example only, a section of the patient's leg is depicted. Fig. 3b) shows the corresponding fluorescence image 7 determined by measuring the fluorescence signal of the fluorescence agent 8, which has been applied to the patient's tissue in the leg. A high-intensity spot or area of the fluorescence signal is visible. This type of fluorescence image is typically classified a diffuse pattern, which is indicative of a later or severe stage of lymphedema. The different types of lymphedema induced fluorescence patterns will be explained in more detail further below. The observed fluorescence pattern strongly indicates an accumulation of lymph, which is due to a slow lymphatic transport and lymphedema in the patient's leg. Advantageously, the physician 3 can locate the area, in which the slow lymphatic transport takes place by comparing the fluorescence image 7 with the visible light image 5. Furthermore, the physician is provided with the output of the AI model, which is for example a stage of severity of lymphedema and/or a clinical type of the fluorescence pattern.

In Fig. 4, there is the overlay image 9, wherein in addition to the images shown in Figs. 3a) and 3b), stitching of the visible light images 5 and fluorescence images 7 has been performed. An exemplary single visible light image 5 and fluorescence image 7 can also be seen in Fig. 4, it respectively projects between the straight dashed lines shown in the large overlay image 9. By stitching together the visible light images 5 and the fluorescence images 7, the large overlay image 9 showing almost the entire body part 4 of the patient 6 can be provided. The fluorescence signal can be shown in false color so as to clearly distinguish from features of the visible light image 5. Also the overlay image can serve as input to the AI model of the CDSS.

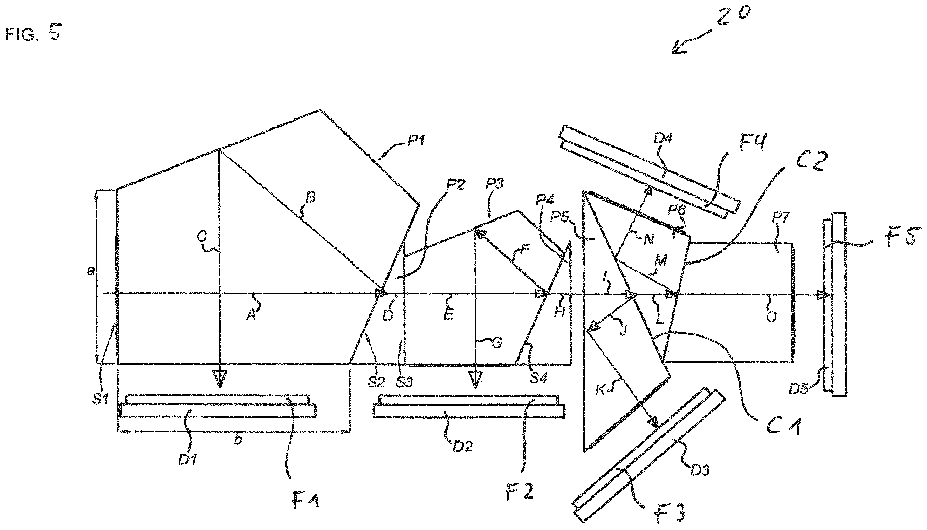

In Fig. 5, there is a prism assembly 20 that can be implemented in the image capturing device 10. A first prism P1 is a pentagonal prism. The incoming light beam A, which is visible light and fluorescence light, enters the first prism P1 via the entrance face S1 and is partially reflected on face S2, being one of the two faces not adjoining the entrance face S1. The reflected beam B is then reflected against a first one of the faces adjoining the entrance face S1. The angle of reflection can be below the critical angle, so that the reflection is not internal (the adjoining face can be coated to avoid leaking of light and reflect the required wavelength of interest). The reflected beam C then crosses the incoming light beam A and exits the first prism P1 through the second one of the faces adjoining the entrance face S1, towards sensor D1. A part of the beam A goes through face S2 and enters compensating prism P2. Two non-internal reflections can be used to direct the incoming beam A via beams B and C towards the sensor D1. Furthermore, there can be no air gaps between prisms P1 and P2 and no air gaps between prisms P3 and P4 and no air gaps between prisms P2 and P3. Prism P2 is a compensator prism which is for adjusting the individual length of the light paths from the entrance face S1 to the sensors D1..D5.

From P2, the beam D enters a second pentagonal prism P3. As in prism P1, inward reflection is used to make the beam cross itself. For brevity, the description of the beam will not be repeated, except to state that in prism P3, the beam parts E, F and G correspond to beam parts A, B and C in prism P1, respectively. Prism P3 can also not use internal reflection to reflect the incoming beam towards sensor D2. Two non-internal reflections can be used to direct the incoming beam E via beams F and G towards sensor D2.

After prism P3, there is another compensating prism P4. Finally, beam H enters the dichroic prism assembly comprising prisms P5, P6, and P7, with sensors D3, D4 and D5 respectively. The dichroic prism assembly is for splitting visible light in red, green and blue components towards respective sensors D3, D4 and D5. The light enters the prism assembly through beam I. Between P5 and P6, an optical coating C1 is placed and between prisms P6 and P7 another optical coating C2 is placed. Each optical coating C1 and C2 has a different reflectance and wavelength sensitivity. At C1, the incoming beam I is partially reflected back to the same face of the prism as through which the light entered (beam J). At that same face, the beam, now labelled K, is once again reflected towards sensor D3. The reflection from J to K is an internal reflection. Thus, sensor D3 receives light reflected by coating C1, and in analogue fashion sensor D4 receives light from beam L reflected by coating S2 (beams M and N), and sensor D5 receives light from beam O that has traversed the prism unhindered.

Between prism P4 and prism P5 there is an air gap. In the prism assembly 20, the following total path lengths can be defined for each endpoint channel (defined in terms of the sensor at the end of the channel):
Sensor D1 (e.g. first near infrared) path: A + B + C
Sensor D2 (e.g. second near infrared) path: A + D + E + F + G
Sensor D3 (e.g. red) path: A + D + E + H + I + J + K
Sensor D4 (e.g. blue) path: A + D + E + H + I + 0
Sensor D5 (e.g. green) path: A + D + E + H + I + M + N

The path lengths are matched, so that A + B + C = A+ D + E + F + G = A + D + E + H + I + J + K = A + D + E + H + I + O = A + D + E + H + I + M + N.

The matching of path lengths can comprise an adjustment for focal plane focus position differences in wavelengths to be detected at the sensors D1 - D5. That is, for example the path length towards the sensor for blue (B) light may not be exactly the same as the path length towards the sensor for red (R) light, since the ideal distances for creating a sharp, focused image are somewhat dependent on the wavelength of the light. The prisms can be configured to allow for these dependencies. D + H lengths can be adjusted and act as focus compensators due to wavelength shifts, by lateral displacement of the compensator prisms P2, P4.

A larger air gap in path I can be used for additional filters or filled with a glass compensator for focus shifts and compensation. An air gap needs to exist in that particular bottom surface of red prism because of the internal reflection in the path from beam J to beam K. A space can be reserved between the prism output faces and each of the sensors D1-D5 to provide an additional filter, or should be filled up with glass compensators accordingly.

The sensors D1 and D2 are IR sensors, configured for capturing the fluorescence image 7. By way of an example, the sensors D1 and D2 plus suitable electronics are a part of the fluorescence imaging unit 22. The sensors D3, D4 and D5 are for capturing the three components of the visible light image 5. By way of an example, the sensors D3, D4 and D5 plus suitable electronics are a part of the visible light imaging unit 24. It is also possible to consider the corresponding prisms that direct the light beams on the sensors, a part of the respective unit, i.e. the fluorescence imaging unit 22 and the visible light imaging unit 24, respectively.

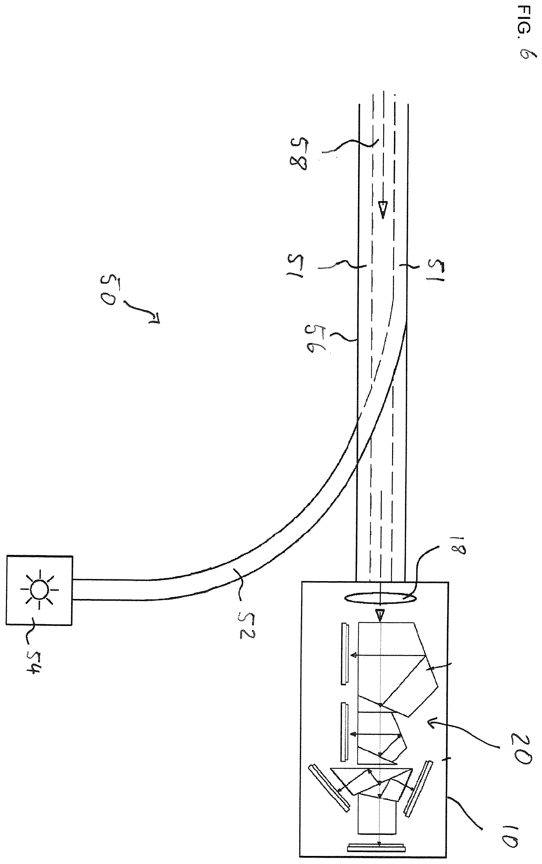

Fig. 6 schematically shows an endoscope 50 or laparoscope. The differences between laparoscopes and endoscopes are relatively small, when considering the aspects of the invention. Hence, where the description mentions an endoscope, a laparoscope configuration is usually also possible. By way of an example only, in the following, reference will be made to an endoscope 50.

The endoscope 50 can serve as a input interface of the CDSS. The endoscope 50 comprises an image capturing device 10 that has been explained in further detail above and which captures a fluorescence image that can be an input feature of the AI model. The image capturing device 10 comprises an objective lens 18 through which the fluorescent light image 7 and the visible light image 5 are captured. The objective lens 18 focuses the incoming light through the entrance face S1 of the prism assembly 20 on the sensors D1 to D5. The objective lens 18 can also be integrated in the last part of the endoscope part to match the prism back focal length.

The endoscope 50 comprises an optical fiber 52 connected to a light source 54 that couples light into the endoscope 50. The light source 54 can provide white light for illumination of the surface 11 of the body part 4 and for capturing of the visible light image 5. Furthermore, the light source 54 can be configured to emit excitation light which is suitable to excite the fluorescent dye that is applied as the fluorescent agent to emit fluorescence light. In other words, the light source 54 can be configured to emit both, visible light and light in the IR spectrum.

Inside a shaft 56 of the endoscope 50, the optical fiber 52 splits off into several fibers 51. The endoscope 50 can have a flexible shaft 56 or a rigid shaft 56. In a rigid shaft 56, a lens system consisting of lens elements and/or relay rod lenses can be used to guide the light through the shaft 56. If the endoscope 50 has a flexible shaft 56 the fiber bundle 51 can be used for guiding the light of the light source 54 to the tip of the endoscope shaft 56. For guiding light from the distal tip of the endoscope shaft 56 (is not shown in Fig. 6) coming from a field of examination to the image capturing device 10 at the proximal end of the shaft 56, a fiber bundle 58 is arranged in the shaft 56 of the endoscope 50. In another embodiment, which is not shown in the figure, the entire image capturing device 10 can be miniaturized and arranged at a distal tip or end of the endoscope shaft 56.

Fig. 7 shows a flowchart of the stitching algorithm, which can be used for stitching of the visible light images and the fluorescence images. The flow chart is more or less self-explanatory and will be very briefly described. Firstly, the acquired series of images (S1) is forwarded to the stitching unit 24 of the processing device 12. The algorithm then performs a frame preselection (step S2). In this preselection step, frames suitable for stitching are selected. S3 represents the selected images to be stitched, they then undergo preprocessing (step S4). In the preprocessed images (S5) a feature extraction is performed (step S6). When the image features have been extracted (S7), image matching (step S8) is performed using the images known from S3 and the extracted features from step S7. Based on the selected images (S9) a transformation of the images is estimated (step S10). This estimate of image transformation (S11), also referred to as stitching parameters, is applied (step S12). The application of the transformation results in transformed images (S13). A further image correction can be performed, for example an exposure correction (step S14). The transformed and corrected images (S15) are stitched together by locating seams (step S16), i.e. lines along which the images are joined together. The data indicating the location of the seams (S17) is used together with the transformed and corrected images (S12) to create a composition of images (step S18). In the given embodiment, this results in the large visible light image or the large fluorescence image, as the stitching results (S19).

Furthermore, the image capturing device 10, which is applied for capturing the visible light images 5 and the fluorescence images 7 can further comprise a measurement unit 32 which is configured to measure a distance d (see Fig. 1) between the surface 11 of the patient's body part 4, which is captured in the visible light image 5, and the image capturing device 10. The measurement unit 32 comprises a distance sensor 33 for that purpose, which communicates with the measurement unit 32, this is for example an ultrasonic sensor, a laser distance sensor or any other suitable distance measurement device. Furthermore, the image capturing device 10 is configured to output a signal, which is indicative of the measured distance d. For example, the image capturing device 10 outputs an optical or acoustical signal giving the operator of the device 10 information on a best distance d for performance of the measurement. Performing the measurement with constant distance d significantly enhances the measurement results, because there is inter alia a homogeneous illumination.

In addition to the distance sensor 33, the image capturing device 10 can include an internal measurement unit (IMU) 35, which can be used to collect data about a rotation in pitch, yaw, and roll as well as acceleration data in the three spatial axes (x, y and z). This information of the IMU may be used as additional data to enhance the performance of the stitching algorithm or to provide feedback to the operator to position and rotate the camera, providing better images for the stitching algorithm.

The processing device 12 can further comprise an AI unit 60 implementing the CDSS. Fig. 8 schematically illustrates the computer based clinical decision support system (CDSS) 62. The CDSS comprises an input interface 64, a processor 66 implementing the AI model 68, and an output interface 70. The input interface 64 is coupled to the image capturing and processing device 2, which serves as a direct data link providing input for the AI model 68. Furthermore, the input interface 64 is coupled to a user input interface 72, which can be a keyboard, touchpad or any other device suitable for that purpose. The input interface 64 is further coupled into the database 74 holding patient related data and in particular an electronic patient record. The output interface 70 is coupled to the display 14, which can be identical to the display 14 of the image capturing and processing device 2, which is shown in Fig. 1.

The exemplary CDSS 62 is configured to provide the classification of the lymphedema induced fluorescence pattern based on a fluorescence image 7. In the various embodiments that have also been outlined above, the CDSS 62 includes as the input interface 64 through which the fluorescence image 7 which is specific to the patient 6 is provided as an input feature to the artificial intelligence (Al) model 68, the image capturing and processing device 2, in particular the image capturing device 10. The processor 66 performs the inference operation in which the fluorescence image 7, a large fluorescence image or an overlay image 9 are applied to the AI model 68 to generate the classification of the lymphedema induced fluorescence pattern. The user interface (III) through which the classification of the lymphedema induced fluorescence pattern, for example a stage of severity of lymphedema and a clinical type of the fluorescence pattern is communicated to a user, e.g., a clinician, can be the display 14.

In some embodiments, the input interface 64 may be a direct data link between the CDSS 62 and one or more medical devices that generate at least some of the input features. For example, the input interface 64 may transmit the fluorescence image directly to the CDSS 62 during a therapeutic and/or diagnostic medical procedure. This can be performed by the image capturing and processing device 2. Additionally, or alternatively, the input interface 64 may be a classical user interface that facilitates interaction between a user and the CDSS 62. For example, the input interface 64 may facilitate a user input interface 72 through which the user may manually enter further patient related data like for example sex, age or a body mass index. Additionally, or alternatively, the input interface 64 may provide the CDSS 62 with access to an electronic patient record from which one or more input features may be extracted. This can be performed by the direct link to the database 74 holding the respective information. In any of these cases, the input interface 64 is configured to collect one or more of the following input features in association with a specific patient 6 on or before a time at which the CDSS 62 is used to assess a level or stage of lymphedema: a fluorescence image 7, a large fluorescence image, an overlay image 9 and optionally additional information from a patient record as mentioned above.

Based on one or more of the above input features, the processor 66 performs an inference operation using the AI model 68 to generate the classification of the lymphedema induced fluorescence pattern. For example, input interface 64 may deliver the fluorescence image 7 into an input layer of the AI model 68 which propagates these input features through the AI model 68 to an output layer. The AI model 68 can provide a computer system the ability to perform tasks, without explicitly being programmed, by making inferences based on patterns found in the analysis of data. AI model 68 explores the study and construction of algorithms (e.g., machine-learning algorithms) that may learn from existing data and make predictions about new data. Such algorithms operate by building an AI model from example training data in order to make data-driven predictions or decisions expressed as outputs or assessments. Enhancement and training of the AI model can also be performed by manual user input correcting for example the automatically generated stage of severity of lymphedema or a clinical type of the fluorescence pattern.

Training of the AI model can also be performed in a cloud using data from various locations. A trained network can then be downloaded and used for a respective procedure. Using a cloud for training is advantageous especially for having higher performance compared to a local system. For security reasons, the system is disconnected from the cloud during the medical procedure.

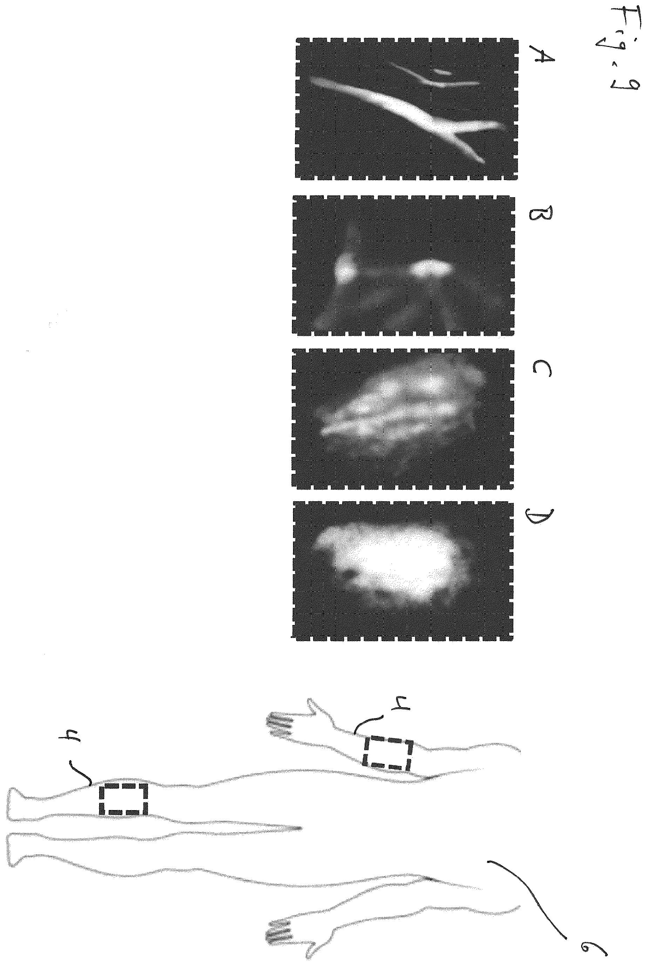

In Fig. 9, there are examples of clinical types of fluorescence patterns. Fig 9 A illustrates a typical linear pattern. This pattern can be observed when the lymphatic system is in good condition and the fluorescent agent 8 is transported together with the lymphatic fluid up the lymphatic vessels. The staging of this finding will typically be stage 1, which means normal lymphatic flow and no signs or symptoms. In Fig. 9 B, there is a splash pattern that can be observed when the lymphatic flow is partly obstructed. Typically, stage 2 will be assigned to this finding, which means there is an accumulation of lymphatic fluid with possible swelling. Fig. 9 C illustrates a stardust pattern which can be observed when lymphedema further progresses. This pattern is typically assigned stage 2 or 3 in which also a permanent swelling of the body part 4 can be observed. In lymphedema stage 3, this swelling does typically not resolve with elevation of the affected body part 4. Finally, Fig. 9 D illustrates a diffuse pattern that is indicative of severe lymphedema. The fluorescent agent 8 together with the lymphatic fluid pours throughout the skin and subcutaneous tissue of the patient 6. This clinical finding will typically be identified with stage 3 or 4, which means there is skin thickening with the growth and deformation of the affected limb 4. The illustrated patterns A to D can be found anywhere on a body or limb 4 of a patient 6 as it is illustrated on the right side of Fig. 9 by rectangle in dashed line.

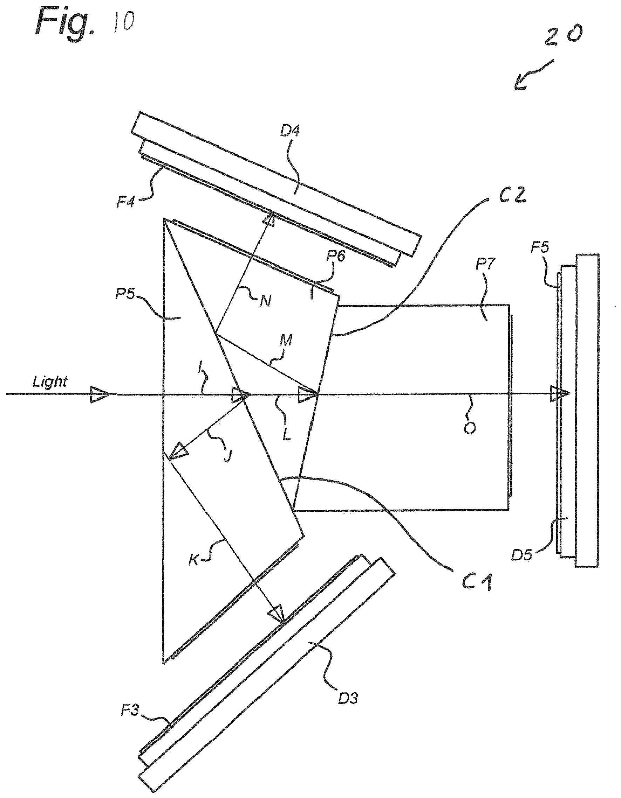

In Fig. 10, there is an embodiment of another prism assembly 20 of the image capturing device 10. The prism assembly 20 comprising prisms P5, P6, and P7, which, for example, are configured for splitting light in red, green and blue components towards respective sensors D3, D4, and D5. According to a further embodiment, the prism assembly 20 is configured to split incoming light in a green component, a red/blue component and an infrared component and to direct these towards the respective sensors D3, D4, and D5. According to still another embodiment, the prism assembly 20 is configured to split incoming light in a visible light component, which is directed to a red/green/blue sensor (RGB sensor), a first infrared component of a first wavelength or wavelength interval and a second infrared component of a second wavelength or wavelength interval, and to direct these towards the respective sensors D3, D4, and D5.

The light enters the prism assembly 20 through the arrow indicated. Between P5 and P6, an optical coating C1 is placed and between prisms P6 and P7 an optical coating C2 is placed, each optical coating C1 and C2 having a different reflectance and wavelength sensitivity. At C1, the incoming beam I is partially reflected back to the same face of the prism P5 as through which the light entered (beam J). At that same face, the beam, now labelled K, is once again reflected towards filter F3 and sensor D3. The reflection from J to K is an internal reflection. Thus, filter F3 and sensor D3 receive light reflected by coating C1, and in analogue fashion filter F4 and sensor D4 receive light from beam L reflected by coating S2 (beams M and N). Filter F5 and sensor D5 receives light from beam O that has traversed the prisms unhindered.

When making reference to the embodiment in which the incoming light is split up in a red, green and blue component, the coatings and filters are selected accordingly.

In the embodiment, in which the incoming light is separated in a green component, a red/blue component and an infrared component, the filter F3 can be a patterned filter (red/blue). In particular, there is an array of red and blue filters in an alternating pattern. The pattern can consist of groups of 2x2 pixels, which are filtered for one particular color. Filter F4 can be a green filter, which means the filter comprises only green filters. There is a single pixel grid with the light received at each pixel being filtered with a green filter. Filter F5 can be an IR filter. Each pixel is filtered with an IR filter.

In general, the coatings C1, C2 should match the filters F3, F4, F5. For example, the first coating C1 may transmit visible light while reflecting IR light, so that IR light is guided towards IR filter F3. The second coating C2 may be transparent for green light while reflecting red and blue light, so that filter F4 should be the red/blue patterned filter and F5 should be the green filter 23.

According to the further embodiment, in which incoming light is split up in the visible light component (RGB), the first infrared component and the second infrared component, the coatings C1, C2 and the filters F3, F4, F5 are configured in that for example the sensor D4 is a color sensor (RGB sensor) for detecting the visible light image in all three colors. Furthermore, the sensor D3 can be configured for detecting fluorescence light of the first wavelength and the sensor D5 is configured for detecting fluorescence light of the second wavelength.

Similarly, when making reference to the prism assembly 20 in Fig. 5, the coatings S1, S2, S3, S4, C1 and C2 as well as the filters F1, F2, F3, F4 and F5, which are arranged in front of a respective one of the sensors D1, D2, D3, D4 and D5, can be configured in that up to four fluorescence light wavelengths can be detected. For example, the sensor D4 is a color sensor for detecting the visible light image in all three colors. The sensor D3 is for detecting fluorescence light of a first wavelength, the sensor D5 is for detecting fluorescence light of a second wavelength, the sensor D1 is for detecting fluorescence light of a third wavelength, the sensor D2 is for detecting fluorescence light of a fourth wavelength.

### Further embodiments:

Embodiment 15: A method of diagnosing lymphedema, comprising the steps of:
   - administering a fluorescent agent (8) to a body part (4) of a patient,
   - determining a classification of a lymphedema induced fluorescence pattern using a computer based clinical decision support system (CDSS) (62), wherein the classification is based on a fluorescence image (7) that is determined by measuring a fluorescence signal in a tissue of the body part (4), to which a fluorescent agent (8) has been added, the method further comprising:
      receiving the fluorescence image (7), which is specific to a patient (6), through an input interface (64) as an input feature of an artificial intelligence (Al) model (68),
      performing an inference operation by a processor (66), in which the fluorescence image (7) is applied to the AI model (68) to generate the classification of a lymphedema induced fluorescence pattern, and
      deriving a diagnostic result from the classification of a lymphedema induced fluorescence pattern, in particular a diagnostic result relative to a stage of lymphedema,
      communicating the classification of a lymphedema induced fluorescence pattern and the diagnostic result to a user through a user interface (UI) (14).
Embodiment 16: A method of long-term therapy of lymphedema, comprising the steps of:
   - diagnosing a severity of lymphedema by performing the method of diagnosing lymphedema according to embodiment 15 on a patient,
   - performing a therapy on the patient, the therapy being customized to the diagnostic result relative to the severity of lymphedema,
   - repeating the steps of diagnosing the severity of lymphedema and performing of the therapy on the patient, wherein in each iteration, the therapy is adjusted to the detected stage of lymphedema, in particular to the detected stage of lymphedema.

All named characteristics, including those taken from the drawings alone, and individual characteristics, which are disclosed in combination with other characteristics, are considered alone and in combination as important to the invention. Embodiments according to the invention can be fulfilled through individual characteristics or a combination of several characteristics. Features which are combined with the wording "in particular" or "especially" are to be treated as preferred embodiments.

### List of References

- 2: image capturing and processing device
- 3: physician
- 4: body part
- 5: visible light image
- 6: patient
- 7: fluorescence image
- 8: fluorescent agent
- 9: overlay image
- 10: image capturing device
- 11: surface
- 12: processing device
- 14: display
- 16: illumination unit
- 18: objective lens
- 20: prism assembly
- 22: fluorescence imaging unit
- 24: visible light imaging unit
- 26: data link
- 28: stitching unit
- 30: superimposing unit
- 32: measurement unit
- 33: distance sensor
- 35: internal measurement unit
- 50: endoscope
- 52: optical fiber
- 51: fibers
- 54: light source
- 56: shaft
- 58: fiber bundle
- 60: AI unit
- 62: CDSS
- 64: input interface
- 66: processor
- 68: AI model
- 70: output interface
- 72: user input interface
- 74: database

- P1: first pentagonal prism
- P2, P4: compensating prism
- P3: second pentagonal prism
- P5, P6, P7: dichroic prism assembly
- A: incoming light beam
- B..O: light beams
- S1: entrance face
- D1..D5: sensors
- C1, C2: coating
- F1..F5: filter

- L: longitudinal direction
- d: distance

## Claims

1. A computer based clinical decision support system (CDSS) (62) that is configured to output a classification of a lymphedema induced fluorescence pattern based on a fluorescence image (7) that is determined from a measurement of a fluorescence signal in a tissue of a body part (4), to which a fluorescent agent (8) has been added, the CDSS (62) comprising:
an input interface (64) through which the fluorescence image (7), which is specific to a patient (6), is provided as an input feature to an artificial intelligence (AI) model (68),
a processor (66), which performs an inference operation in which the fluorescence image (7) is applied to the AI model (68) to generate the classification of a lymphedema induced fluorescence pattern, and
a user interface (III) (14) through which the classification of the lymphedema induced fluorescence pattern is communicated to a user.

2. The CDSS (62) according to claim 1, wherein the classification of a lymphedema induced fluorescence pattern is a stage of severity of lymphedema and/or a clinical type of the fluorescence pattern.

3. The CDSS (62) according to claim 1 or 2, wherein the fluorescence image (7) and a corresponding visible light image (5) are provided through the input interface as input features to the artificial intelligence (AI) model.

4. The CDSS (62) according to claim 3, wherein the input interface (64) is a direct link to an image capturing and processing device (2) that is configured to measure the fluorescence signal in the tissue of the body part (4) and that is configured to image a surface (11) of the body part (4), wherein the tissue to which the fluorescent agent (8) has been added forms part of the body part (4), the image capturing and processing device (2) comprising an image capturing device (10), which comprises:
- an illumination unit (16) configured to illuminate the tissue with excitation light having a wavelength suitable to generate emitted light by excited emission of the fluorescent agent (8),
- a fluorescence imaging unit (22) configured to capture the fluorescence image by spatially resolved measurement of the emitted light so as to provide the fluorescence image,
- a visible light imaging unit (24) configured to capture the corresponding visible light image of a section of a surface (11) of the body part (4), wherein the fluorescence imaging unit (22) and the visible light imaging unit (24) are configured in that a viewing direction and/or a perspective of the fluorescence image and the corresponding visible light image are linked via a known relationship.

5. The CDSS (62) according to claim 4, wherein a large fluorescence image and a corresponding large visible light image are provided through the input interface (64) as input features to the artificial intelligence (AI) model (68), and wherein
- the fluorescence imaging unit (22) and the visible light imaging unit (24) are further configured to repeat capturing of the fluorescence image and the visible light image to provide a series of fluorescence images and a series of visible light images,
the image capturing and processing device (2) further comprises a processing device 12, which comprises:
- a stitching unit (28) configured to apply a stitching algorithm on the series of visible light images to generate the large visible light image of the body part (4), the stitching algorithm determining and applying a set of stitching parameters,
wherein the stitching unit (28) is further configured to apply the stitching algorithm on the series of fluorescence images (7) to generate the large fluorescence image, wherein the stitching algorithm applies the set of stitching parameters determined when performing the stitching of the visible light images (5).

6. The CDSS (62) according to any of claims 3 to 5, wherein the image capturing device (2) comprises a dichroic prism assembly configured to receive fluorescent light and visible light through an entrance face, the prism assembly comprising: a first prism (P1), a second prism (P3), a first compensator prism (P2) located between the first prism (P1) and the second prism (P3),
a further dichroic prism assembly (P5, P6, P7) for splitting the visible light in three light components, and a second compensator prism (P4) located between the second prism (P3) and the further dichroic prism assembly (P5, P6, P7),
wherein the first prism (P1) and the second prism (P3) each have a cross section with at least five corners, each corner having an inside angle of at least 90 degrees, wherein the corners of the first prism (P1) and the second prism (P3) each have a respective entrance face (S1, S2) and a respective exit face, and are each designed so that an incoming beam which enters the entrance face of the respective prism in a direction parallel to a normal of said entrance face is reflected twice inside the respective prism and exits the respective prism through its exit face parallel to a normal of said exit face,
wherein the normal of the entrance face and the normal of the exit face of the respective prism are perpendicular to each other; and
wherein, when light enters the first prism (P1) through the entrance face, the light is partially reflected towards the exit face of the first prism (P1) thereby traveling a first path length from the entrance face of the first prism (P1) to the exit face of the first prism (P1 ), and the light partially enters the second prism (P3) via the first compensator prism (P2) and is partially reflected towards the exit face of the second prism (P3), thereby traveling a second path length from the entrance face of the first prism (P1) to the exit face of the second prism (P3),
wherein the first prism (P1) is larger than the second prism (P3) so that the first and the second path lengths are the same.

7. The CDSS (62) of any of the preceding claims, wherein the input interface (64) further is a direct link to an electronic patient record, wherein patient related data, in particular data relative to: age, gender, height, weight, BMI (Body Mass Index), fat mass, muscle mass, daily exercise mass, presence or absence of work, skin color, medication status, presence or absence of vascular disease, in particular varicose veins or venous edema, presence or absence of disease, in particular dialysis or diabetes, amount of albumin in blood, kidney function, liver function, heart function, Hemoglobin concentration in the blood, blood estimate, lipid metabolism, blood glucose concentration in the blood, urea or nitrogen, ABI (ankle/humeral index) value; lymphatic function measurement data at the same location (40 A) before the occurrence of lymphaedema, endocrine information, hormone level of the patient, are provided through the input interface as further input features to the artificial intelligence (AI) model (68).

8. Computer implemented method of determining a classification of a lymphedema induced fluorescence pattern using a computer based clinical decision support system (CDSS) (62), the classification being based on a fluorescence image (7) that is determined by measuring a fluorescence signal in a tissue of a body part (4), to which a fluorescent agent (8) has been added, the method comprising:
receiving the fluorescence image (7), which is specific to a patient (6), through an input interface (64) as an input feature of an artificial intelligence (AI) model (68),
performing an inference operation by a processor (66), in which the fluorescence image (7) is applied to the AI model (68) to generate the classification of a lymphedema induced fluorescence pattern, and
communicating the classification of a lymphedema induced fluorescence pattern to a user through a user interface (III) (14).

9. The method according to claim 8, wherein the classification of a lymphedema induced fluorescence pattern is a stage of severity of lymphedema and/or a clinical type of the fluorescence pattern.

10. The method according to claim 8 or 9, wherein the fluorescence image (7) and a corresponding visible light image (5) are provided through the input interface as input features to the artificial intelligence (AI) model (68).

11. The method of claim 10, wherein the input interface receives the fluorescence image (7) and the corresponding visible light image (5) through a direct link to an image capturing and processing device (2) that is configured to measure the fluorescence signal in the tissue of the body part (4) and that is configured to image a surface (11) of the body part (4), wherein the tissue to which the fluorescent agent (8) has been added forms part of the body part (4), and wherein the image capturing and processing device (2) comprises an image capturing device (10), which comprises an illumination unit (16), a fluorescence imaging unit (22) and a visible light imaging unit (24), the method further comprising the steps of:
- illuminating of the tissue by the illumination unit (16) with excitation light having a wavelength suitable to generate emitted light by excited emission of the fluorescent agent (8),
- capturing of the fluorescence image by the fluorescence imaging unit (22) by spatially resolved measurement of the emitted light so as to provide the fluorescence image,
- capturing of the visible light image by the visible light imaging unit (24) by capturing the corresponding visible light image of a section of a surface (11) of the body part (4), wherein the fluorescence imaging unit (22) and the visible light imaging unit (24) are configured in that a viewing direction and/or a perspective of the fluorescence image and the corresponding visible light image are linked via a known relationship.

12. The method according to claim 11, wherein a large fluorescence image and a corresponding large visible light image are provided through the input interface (64) as input features to the artificial intelligence (AI) model (68), and wherein
- the fluorescence imaging unit (22) and the visible light imaging unit (24) repeat capturing of the fluorescence image (7) and the visible light image (5) to provide a series of fluorescence images and a series of visible light images, wherein
the image capturing and processing device (2) further comprises a processing device, which comprises a stitching unit (28) and the method further comprises the steps of:
- applying a stitching algorithm on the series of visible light images (5) by the stitching unit (28) to generate the large visible light image of the body part (4), the stitching algorithm determining and applying a set of stitching parameters,
further applying the stitching algorithm on the series of fluorescence images (7) by the stitching unit (28) to generate the large fluorescence image, wherein the stitching algorithm applies the set of stitching parameters determined when performing the stitching of the visible light images (5).

13. The method according to any of claims 8 to 12, wherein the measurement of the fluorescence signal is performed on a tissue, to which at least a first and a second fluorescent agent (8) has been added, wherein the step of capturing the fluorescence image comprises:
- capturing a first fluorescence image in a first wavelength range, which is generated by illuminating the tissue with first excitation light having a first wavelength suitable to generate emitted light by a first excited emission of the first fluorescent agent (8), and
- capturing a second fluorescence image in a second wavelength range, which is generated by illuminating the tissue with second excitation light having a second wavelength suitable to generate emitted light by a second excited emission of the second fluorescent agent (8), and wherein
the first and the second fluorescence are provided through the input interface as input features to the artificial intelligence (AI) model, wherein
the input interface (64) receives the first and the second fluorescence image as an input features of the artificial intelligence (AI) model (68), and
the processor (66) performs the inference operation by applying the first and the second fluorescence image to the AI model (68) to generate the classification of the lymphedema induced fluorescence pattern.

14. The method of any of claims 8 to 13, wherein patient related data, in particular data relative to age, gender, height, weight, BMI (Body Mass Index), fat mass, muscle mass, daily exercise mass, presence or absence of work, skin color, medication status, presence or absence of vascular disease, in particular varicose veins or venous edema, presence or absence of disease, in particular dialysis or diabetes, amount of albumin in blood, kidney function, liver function, heart function, Hemoglobin concentration in the blood, blood estimate, lipid metabolism, blood glucose concentration in the blood, urea or nitrogen, ABI (ankle/humeral index) value; lymphatic function measurement data at the same location (40 A) before the occurrence of lymphaedema, endocrine information, hormone level of the patient, is provided through the input interface as further input features to the artificial intelligence (AI) model (68), via a direct link to an electronic patient record.
